**Europäisches Patentamt**

**European Patent Office**

**Office européen des brevets**

(19)

(11) Veröffentlichungsnummer : **0 434 088 B1**

(12)
# EUROPÄISCHE PATENTSCHRIFT

(45) Veröffentlichungstag der Patentschrift :
**26.05.93 Patentblatt 93/21**

(51) Int. Cl.$^5$ : **A61K 31/205**

(21) Anmeldenummer : **90125138.9**

(22) Anmeldetag : **21.12.90**

(54) **L-Carnitin-haltige Zubereitungen.**

(30) Priorität : **22.12.89 CH 4633/89**

(43) Veröffentlichungstag der Anmeldung :
**26.06.91 Patentblatt 91/26**

(45) Bekanntmachung des Hinweises auf die
Patenterteilung :
**26.05.93 Patentblatt 93/21**

(84) Benannte Vertragsstaaten :
**AT BE CH DE FR GB IT LI NL SE**

(56) Entgegenhaltungen :
**EP-A- 0 150 688**
**DD-A- 93 347**
**GB-A- 2 058 566**
**US-A- 4 537 772**
**DERWENT FILE SUPPLIER WPI, 1966, AN-**
**=66-09976F, Derwent Publications Ltd, Lon-**
**don, GB**

(73) Patentinhaber : **LONZA AG**
**Gampel/Wallis (CH)**

(72) Erfinder : **Kohl, Willibald F., Dr. Dipl.-Ing.**
**Gurtenweg 29A**
**Muri bei Bern (Kanton Bern) (CH)**
Erfinder : **Scholl, Thomas, Dr.**
**Terbinerstrasse 40**
**Visp (Kanton Wallis) (CH)**

(74) Vertreter : **Weinhold, Peter, Dr. et al**
**Patentanwälte Dipl.-Ing. G. Dannenberg Dr. P.**
**Weinhold Dr. D. Gudel Dipl.-Ing. S. Schubert**
**Dr. P. Barz Siegfriedstrasse 8**
**W-8000 München 40 (DE)**

## Beschreibung

Die Erfindung betrifft L-Carnitin-haltige Präparate zur oralen Anwendung in Tabletten-, Kapsel- oder Pulverform.

L-Carnitin spielt eine wichtige Rolle beim Fettstoffwechsel und wird vor allem in der Sporternährung, aber auch zur Behandlung von Krankheiten mit Stoffwechselstörungen eingesetzt. In der Sporternährung finden L-Carnitin-haltige Präparate eine breite Anwendung, da sie wesentlich zur Versorgung der Muskeln mit Energie beitragen und die Ausdauerleistung fördern. Derartige Präparate haben eine grosse Bedeutung, da sie die Muskeltätigkeit verbessern, und dadurch eine erhöhte Ausdauer und Stresstoleranz, sowie verzögerte Ermüdung und kürzere Erholungszeit bewirken.

Die Verwendung L-Carnitin-haltiger Präparate beschränkt sich jedoch nicht auf die Sporternährung, sondern diese können auch für geriatrische Zwecke und als allgemeine Ernährungszusätze verwendet werden.

Die Applikation kann dabei grundsätzlich sowohl enteral als auch parenteral erfolgen. Für die bevorzugte enterale, d. h. orale Applikation sind dabei geeignete Darreichungsformen, vorzugsweise in Tabletten- oder Kapselform, gegebenenfalls auch in Pulver- oder Granulatform, erforderlich. Die Herstellung erfolgt dabei nach Methoden der pharmazeutischen Technologie, unabhängig davon, ob die Darreichungsform Ernährungszwecken oder therapeutischen Zwecken dienen soll. Die Herstellung und Handhabung solcher Darreichungsformen wurde bisher durch die hohe Hygroskopizität von L-Carnitin wesentlich erschwert. So müssen beispielsweise Tabletten, die L-Carnitin enthalten, unter Feuchtigkeitsausschluss hergestellt und einzeln luftdicht verpackt werden, da sie schon bei normaler Luftfeuchtigkeit in kurzer Zeit zerfliessen würden. Ausserdem enthält L-Carnitin oft Spuren von Trimethylamin, die wegen des fischartigen Geruchs abstossend auf den Anwender wirken.

Aufgabe der Erfindung war daher, eine nicht hygroskopische und geruchlose Form von L-Carnitin bereitzustellen, die keine physiologisch bedenklichen Zusätze enthält und sich insbesondere zur Herstellung von Tabletten oder Kapseln bevorzugt eignet.

Erfindungsgemäss wird diese Aufgabe gemäss Patentanspruch 1 durch die Verwendung von L-Carnitin-L-tartrat gelöst. Unter L-Carnitin-L-tartrat ist hierbei das Salz von L-Carnitin mit L-Weinsäure im molaren Verhältnis 2:1 zu verstehen.

Es wurde gefunden, dass L-Carnitin-L-tartrat bei normaler Luftfeuchtigkeit ($\leqq$ 60% rel. Feuchte) lagerstabil ist und ohne besondere Vorkehrungen verarbeitet werden kann. L-Carnitin-L-tartrat bildet ein kristallines Pulver, das sich leicht verarbeiten lässt und sich insbesondere zur Verarbeitung mit schnell-laufenden Maschinen eignet, da es weder zur Verklumpung noch zur Verklebung neigt. Ausserdem ist es völlig geruchlos und hat durch die gebundene Weinsäure einen erfrischenden säuerlichen Geschmack.

Das L-Carnitin-L-tartrat wird vorteilhaft allein oder mit weiteren Wirkstoffen, wie beispielsweise Vitaminen, Aminosäuren, Spurenelementen oder Mineralstoffen, sowie gegebenenfalls für die jeweilige Darreichungsform üblichen Hilfsstoffen verwendet. Die Darreichungsformen umfassen insbesondere alle Arten von Tabletten, sowohl solche, die unzerkaut geschluckt werden, als auch Kau- oder Lutschtabletten, sowie solche, die vor der Einnahme in einer Flüssigkeit aufgelöst werden. Dazu zählen einerseits nicht überzogene Tabletten, sowohl in einschichtiger als auch in mehrschichtiger oder ummantelter Form sowie Brausetabletten, andererseits überzogene Tabletten, wie Filmtabletten oder Dragees. Weitere bevorzugte Darreichungsformen sind Kapseln aus Weich- oder Hartgelatine. Hiervon sind Hartgelatinekapseln in Form von Steckkapseln besonders bevorzugt. Weiterhin kann L-Carnitin-L-tartrat vorteilhaft als Pulver, beispielsweise mit gasentwickelnden Zusätzen als Brausepulver, oder als Granulat verwendet werden. Hilfsstoffe sind beispielsweise Füllstoffe, Bindemittel, Gleit- und Formtrennmittel, Fliessreguliermittel und Sprengmittel für die Tablettenherstellung, sowie Farb- und Aromastoffe. Solche Hilfsstoffe sind dem Fachmann bekannt, ebenso ihre Anwendung sowie die Technik der Herstellung der genannten Darreichungsformen.

Die Wirkstoffmenge pro Dosierungseinheit liegt zweckmäßigerweise zwischen 50 mg und 1000 mg, vorzugsweise zwischen 100 mg und 500 mg (berechnet als L-Carnitin).

Als für die Tablettenherstellung erforderliche Hilfsstoffe können eingesetzt werden:

Füllstoffe, z.B. Lactose, Saccharose, Fructose, Mannit, andere Zucker, Zuckeralkohole und Stärke;

Bindemittel, z.B. Stärke, Gelatine, Celluloseether, Cellulose, Carboxymethylcellulose, Polyvinylpyrrolidon und andere wasserlösliche Polymere;

Sprengmittel, z.B. Stärke, Stärkeether, Alginate, Celluloseester, Celluloseether und Pektinderivate;

Gleit- und Formtrennmittel, z.B. Talkum, Stearate, hochdisperses Siliciumdioxid und Silikone; sowie wahlweise Lebensmittelfarbstoffe, Aromen, Vitamine, Aminosäuren, Mineralien und Spurenelemente, Süßstoffe und Natriumhydrogencarbonat im Falle von Brausetabletten.

Bevorzugt ist ein Anteil von L-Carnitin-L-tartrat (berechnet als L-Carnitin) von 20 bis 70% des totalen Tablettengewichtes.

Pulver zur Verwendung in Kapseln oder zur direkten oralen Verabreichung bestehen aus L-Carnitin-L-tartrat und Fließregulierungsmittel, wie z.B. hochdispersem Siliciumdioxid, Talkum und Stearaten. Granulate zur Verwendung in Kapseln oder zur direkten oralen Verabreichung bestehen aus L-Carnitin-L-tartrat und Bindemitteln. Die Mengen der Fließregulierungsmittel bzw. Bindemittel liegen sinnvollerweise zwischen 0,1 Gew. % und 10 Gew.-%, vorzugsweise zwischen 0,5 Gew.-% und 5 Gew.-%.

Bei der erfindungsgemäßen Verwendung von L-Carnitin-L-tartrat ist die Menge der zur Herstellung fester oraler Zusammensetzungen benötigten Füllstoffe und Bindemittel allgemein wesentliche geringer als bei der Verwendung von L-Carnitin. So ist es z.B. nicht möglich, lagerfähige Kapseln mit L- Carnitin ohne Verwendung von Füllstoffen herzustellen.

Die nachfolgenden Beispiele verdeutlichen die Ausführung der Erfindung.

Beispiel 1

Herstellung von L-Carnitin-L-tartrat

L-Weinsäure wurde in der erforderlichen Menge von heissem 90%igem wässrigem Ethanol gelöst, die berechnete Menge L-Carnitin zugesetzt, das Salz durch Abkühlen zur Kristallisation gebracht, abfiltriert und getrocknet.

Das Produkt zeigt folgende Eigenschaften:

farblose Kristalle
Schmelzpunkt: 169 - 175°C
$[\alpha]_D^{25}$ : -10,9° $\pm$ 0,60 (25°C, c = 1% in Wasser)
Zusammensetzung: Molverhältnis Carnitin: Weinsäure = 2:1 ($^1$H-NMR)
Wasserlöslichkeit: ca. 73 g/100 g Lösung

Wasseraufnahme bei einer Luftfeuchtigkeit von 32%:

| Nach Stunden | L-Carnitin-tartrat | L-Carnitin |
|:---:|:---:|:---:|
| 1 | 0% | 1,9% |
| 2 | 0% | 3,6% |
| 4 | 0% | 6,3% |
| 8 | 0% | 8,6% |
| 24 | 0% | 12,3% |

Wasseraufnahme bei einer Luftfeuchtigkeit von 66%:

| Nach Stunden | L-Carnitin-L-tartrat | L-Carnitin |
|:---:|:---:|:---:|
| 1 | 0% | 6,0% |
| 2 | 0% | 9,6% |
| 4 | 0% | 21,6% |
| 8 | 0,1% | 45,2% |
| 24 | 0,1% | 67,7% |

Beispiel 2

Lutschtabletten mit Orangen-Aroma

Es wurden Lutschtabletten von 2 200 mg Einzelgewicht gemäss folgender Rezeptur hergestellt :

| | |
|---|---|
| L-Carnitin-L-tartrat | 732 mg |
| Fructose | 1 089 mg |
| Orangen-Aroma | 30 mg |
| Chinolingelb-Lack | 4 mg |
| Carboxymethylcellulose | 25 mg |
| Polyvinylpyrrolidon | 20 mg |
| Sacharosestearat | 100 mg |
| Talcum | 160 mg |
| Magnesiumstearat | 40 mg |

Die Fructose wurde mit der Carboxymethylcellulose und 3/4 des Lackfarbstoffs vermischt, mit einer Lösung des Polyvinylpyrrolidons in 85%igem Ethanol granuliert, getrocknet und gesiebt. Das Orangenaroma und der restliche Lackfarbstoff wurden mit 20% des L-Carnitin-L-tartrats vermischt, gesiebt und anschliessend zu dem restlichen L-Carnitin-L-tartrat gegeben.

Das Saccharosestearat wurde hinzugefügt, sorgfältig eingemischt und die Mischung abschliessend gesiebt.

Schliesslich wurde zunächst das Fructosegranulat und zuletzt der Talk und das Magnesiumstearat eingestreut und jeweils homogen gemischt.

Die Mischung wurde bis zur Tablettierung in einem dichtschliessenden Behälter aufbewahrt und dann zu runden, biplanen Tabletten mit Facettenrand gepresst. Der Tablettendurchmesser betrug 20 mm, die Dicke ca. 5,4 mm. Zum Vergleich wurden gleichartige Lutschtabletten hergestellt, die anstelle des L-Carnitin-L-tartrats 500 mg L-Carnitin und 232 mg mikrokristalline Cellulose (zum Gewichtsausgleich) enthielten.

An zerkleinerten Tabletten wurde jeweils unter konstanter relativer Luftfeuchtigkeit die Wasseraufnahme bestimmt. Bei einer Lagerung bei einer relativen Luftfeuchtigkeit von 56% nahmen die zerkleinerten Tabletten, die L-Carnitin-L-tartrat enthielten, auch nach 10 Tagen kein Wasser auf. Im Vergleich dazu zeigten Tabletten, die L-Carnitin enthielten, unter gleichen Bedingungen eine Wasseraufnahme von 12%. Die erfindungsgemässe Verwendung von L-Carnitin-L-tartrat ergab auch bei extremen Bedingungen lagerfähige Tabletten.

Beispiel 3

Lutschtabletten mit Pfefferminz-Aroma

Es wurden Lutschtabletten von 2200 mg Einzelgewicht gemäss folgender Rezeptur hergestellt:

| L-Carnitin-L-tartrat | 732 mg |
|---|---|
| Mannit | 1100 mg |
| Aspartam | 13 mg |
| Pfefferminz-Aroma | 10 mg |
| Carboxymethylcellulose | 25 mg |
| Polyvinylpyrrolidon | 20 mg |
| Sacharosestearat | 100 mg |
| Talcum | 160 mg |
| Magnesiumstearat | 40 mg |

Der Mannit wurde mit Carboxymethylcellulose und dem Aspartam vermischt, mit einer Lösung von Polyvinylpyrrolidon in 80%-igem Ethanol granuliert, getrocknet und gesiebt.

Das Pfefferminzaroma wurde mit ca. 10% des L-Carnitin-L-tartrats vermischt, gesiebt und anschliessend dem restlichen L-Carnitin-L-tartrat zugesetzt. Das Saccharosestearat wurde hinzugefügt, sorgfältig eingemischt und das Ganze gesiebt.

Anschliessend wurde erst das Mannitgranulat und dann der Talk und das Magnesiumstearat zugegeben und jeweils homogen eingemischt.

Die Mischung wurde bis zur Tablettierung in einem dichtschliessenden Behälter aufgewahrt und dann zu runden, biplanen Tabletten mit Facettenrand gepresst. Der Tablettendurchmesser betrug 20 mm, die Dicke ca. 5,7 mm.

Zum Vergleich wurden wiederum wie in Beispiel 2 Tabletten mit L-Carnitin und mikrokristalliner Cellulose hergestellt. Auch mit diesen Tabletten wurde wie in Beispiel 2 die Wasseraufnahme bestimmt.

Die erfindungsgemässe Verwendung von L-Carnitin-L-tartrat ergab stabile, lagerfähige Tabletten, während Tabletten auf der Basis von L-Carnitin nach einwöchiger Lagerung durch Wasseraufnahme eine klebrige Masse bildete.

Beispiel 4

Tablette zum Schlucken

Gemäss folgender Rezeptur wurden 12 000 Tabletten zu je 650 mg hergestellt:

| L-Carnitin-L-tartrat | 4,392 kg |
|---|---|
| Lactose-Monohydrat, direkttablettierbare | 2,028 kg |
| Weizenstärke | 420 g |
| Cellulose, mikrokristallin | 360 g |
| Siliciumdioxid (Aerosil®200) | 60 g |
| Talcum | 480 g |
| Magnesiumstearat | 60 g |

Das L-Carnitin-L-tartrat wurde mit der Weizenstärke und der Cellulose homogen gemischt und gesiebt. Die Lactose wurde zugefügt, gleichmässig untergemischt und das Gemisch nochmals gesiebt. Talcum, Magnesiumstearat und Siliciumdioxid wurden innig miteinander vermischt, gesiebt und in die Wirkstoffmischung eingestreut. Das gesamte Gemenge wurde nochmals durchgemischt und bis zur Tablettierung in einem dicht verschlossenen Behälter aufbewahrt.

Es wurden kreisrunde Tabletten mit Facettenrand von 13 mm Durchmesser und ca. 3,9 mm Dicke gepresst.

Diese wiesen eine Druckfestigkeit von 60 - 70 N auf und zerfielen in Wasser von 20°C innerhalb von 15 - 17 Minuten.

Beispiel 5

Kapseln zur Verwendung als Nahrungs-Ergänzung (Supplement)

Es wurden Hart-Gelatine-Kapseln mit L-Carnitin-L-tartrat hergestellt, entsprechend der nachfolgenden Zusammensetzung:

L-Carnitin-L-tartrat      366 mg  
Magnesiumstearat      4 mg

Magnesiumstearat wurde mit einem Sieb von 0,5 mm Maschenweite gesiebt, L-Carnitin-L-tartrat wurde zugesetzt, und die beiden Komponenten wurden 15 Min. lang intensiv vermischt.

Danach wurde in CONI-SNAP®-Kapseln der Grösse 1 abgefüllt. Dies ergab Kapseln, die auch bei tropischen Bedingungen lagerfähig waren.

366 mg L-Carnitin-L-tartrat pro Kapsel entsprach einer Menge von 250 mg L-Carnitin.

**Patentansprüche**

1. Verwendung von L-Carnitin-L-tartrat zur Herstellung von L-Carnitin-haltigen oralen Darreichungsformen.

2. Verwendung von L-Carnitin-L-tartrat zur Herstellung von L-Carnitin-haltigen Tabletten, Kapseln, Pulvern oder Granulaten.

3. L-Carnitin-haltige Zubereitung, insbesondere zur enteralen Applikation in Tabletten-, Kapsel-, Pulver- oder Granulatform, gekennzeichnet durch einen Gehalt an L-Carnitin-L-tartrat.

**Claims**

1. Use of L-carnitine-L-tartrate for the preparation of L-carnitine containing compositions for oral administration.

2. Use of L-carnitine-L-tartrate for the preparation of L-carnitine containing tablets, capsules, powders or granulates.

3. L-carnitine containing pharmaceutical compositions, particularly for the enteral administration in the form of tablets, capsules, powders or granulates, characterised in that they contain L-carnitine-L-tartrate.

**Revendications**

1. Utilisation de la L-carnitine-L-tartrate pour la préparation de formes d'administration orale contenant de la L-carnitine.

2. Utilisation de la L-carnitine-L-tartrate pour la préparation de comprimés, gélules, poudres ou granulés contenant de la L-carnitine.

3. Préparation contenant de la L-carnitine, notamment pour l'application entérale sous forme de comprimés, gélules, poudres ou granulés, caractérisée par une teneur en L-carnitine-L-tartrate.